(19) Europäisches Patentamt
European Patent Office
Office européen des brevets

(11) Publication number: **0 049 971 B1**

(12) # EUROPEAN PATENT SPECIFICATION

(45) Date of publication of patent specification: **08.04.87**

(51) Int. Cl.[4]: **C 07 D 237/24,** A 01 N 43/58

(21) Application number: **81304487.2**

(22) Date of filing: **29.09.81**

(54) **Substituted pyridazines, processes for making them, their use as plant growth regulators, and plant growth regulating compositions containing them.**

(30) Priority: **03.10.80 US 193672**
**03.10.80 US 193677**

(43) Date of publication of application:
**21.04.82 Bulletin 82/16**

(45) Publication of the grant of the patent:
**08.04.87 Bulletin 87/15**

(84) Designated Contracting States:
**AT BE CH DE FR GB IT LI NL SE**

(56) References cited:
**FR-A-2 383 605**
**FR-A-2 392 980**

**MONATSHEFTEN DER CHEMIE, vol. 91, 1960 WIEN (AT) E. NÖLKEN et al.: "Synthesen von Heterocyclen, 28. Mitt.: Über Azokörper der Pyronocumarine" page 1162**

**JOURNAL OF ORGANIC CHEMISTRY, vol. 44, no. 17, 1979, American Chemical Society WASHINGTON (US) S. GELIN: "Synthesis and Reactions of Some 5-Hydroxypyridazinium Hydroxide Inner Salts from 3 (2 H)-Furanones" pages 3.053 - 7**

(73) Proprietor: **Rohm and Haas Company**
**Independence Mall West**
**Philadelphia, Pennsylvania 19105 (US)**

(72) Inventor: **Patterson, Dennis Ray**
**407 Washington Avenue**
**North Wales Pennsylvania, 19454 (US)**

(74) Representative: **Angell, David Whilton et al**
**Rohm and Haas Company Patent Department**
**Chesterfield House Bloomsbury Way**
**London WC1A 2TP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns novel substituted pyridazines and their use as plant growth regulators, particularly as chemical sterilants for producing hybrid seed of cereal grain plants; also concerned are processes for making the new compounds and plant growth regulating compositions containing them.

In Belgian Patent specification No. 864,704 and its foreign equivalents: French No. 2,392,980, W. German No. 2,808,795, Egyptian No. 13401, Australian No. 78/33980 and New Zealand No. 186,596 there are described 1-aryl(or substituted aryl)-1,4-dihydro-4-oxo-3-carboxy-6-alkylpyridazines and derivatives thereof. These compounds are disclosed as having utility as plant growth regulators particularly as chemical hybridizing agents for the production of hybrid cereal seed.

The present invention provides novel substituted pyridazines which bear some resemblance to those of the prior art compounds noted above but which are principally characterized in that they contain a carboxy group, or derivative thereof, in the 5-position. Representative compounds of the new substituted pyridazines are unexpectedly safer chemical hybridizing agents as compared with the cited prior art compounds in that they have significantly reduced adverse effects as regards hybrid seed quality or amount of plant injury at doses above those required to produce maximum male sterility thus allowing for higher yields of hybrid seed.

The pyridazine compounds concerned in this invention are those of Formula (I) below.

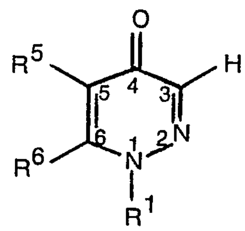

(I)

wherein $R^1$ is $(C_1—C_4)$alkyl, phenyl or naphthyl each of the last two groups being optionally substituted with up to three (preferably one or two) of the same or different substituents selected from halogen (viz. chlorine, bromine, fluorine and iodine, trihalomethyl (e.g. trifluoromethyl or trichloromethyl), $(C_1—C_4)$alkoxy, $(C_1—C_4)$alkyl and cyano;

$R^5$ is a carboxy (—COOH) group or agronomically acceptable alkali metal salt thereof (particularly a salt of Na, K or Li), an alkoxycarbonyl (—COOR) group or a substituted carbamoyl (—CONR'R'') group, wherein R represents $(C_1—C_4)$alkyl, R' represents hydrogen or $(C_1—C_4)$alkyl and R'' represents $(C_1—C_4)$alkyl; and

$R^6$ is a $(C_1—C_4)$alkyl group or an optionally substituted phenyl or naphthyl group as defined for $R^1$ above.

Compounds of Formula (I) meriting particular attention are those wherein $R^1$ is halophenyl, particularly 4-halophenyl, preferably 4-chloro- or 4-bromophenyl; and $R^5$ is $—CO_2Na$ or $CO_2K$ and $R^6$ is methyl or ethyl, preferably ethyl.

The term alkyl used in this specification includes both straight and branched chain alkyl groups and, where unqualified, alkyl means n-alkyl.

Also included within the scope of the invention are agronomically acceptable acid addition salts of compounds of Formula (I). Typical acid addition salts are those formed with strong acids such as hydrochloric, hydrobromic, sulfuric and nitric acids.

Typical compounds of Formula (I)

1-phenyl-1,4-dihydro-4-oxo-6-methylpyridazine-5-carboxylic acid,
1-phenyl-1,4-dihydro-4-oxo-6-ethylpyridazine-5-carboxylic acid,
1-phenyl-1,4-dihydro-4-oxo-6-propylpyridazine-5-carboxylic acid,
1-phenyl-1,4-dihydro-4-oxo-6-butylpyridazine-5-carboxylic acid,
1-methyl-1,4-dihydro-4-oxo-6-methylpyridazine-5-carboxylic acid,
1-phenyl-1,4-dihydro-4-oxo-6-phenylpyridazine-5-carboxylic acid,
1-(4-chlorophenyl)-1,4-dihydro-4-oxo-6-methylpyridazine-5-carboxylic acid,
1-(4-bromophenyl)-1,4-dihydro-4-oxo-6-ethylpyridazine-5-carboxylic acid,
1-(3,4-dichlorophenyl)-1,4-dihydro-4-oxo-6-propylpyridazine-5-carboxylic acid,
1-(4-iodophenyl)-1,4-dihydro-4-oxo-6-butylpyridazine-5-carboxylic acid,
1-(4-fluorophenyl)-1,4-dihydro-4-oxo-6-butylpyridazine-5-carboxylic acid,
1-(4-chlorophenyl)-1,4-dihydro-4-oxo-6-phenylpyridazine-5-carboxylic acid,
1-(3-chlorophenyl)-1,4-dihydro-4-oxo-6-methylpyridazine-5-carboxylic acid,
1-(2-chlorophenyl)-1,4-dihydro-4-oxo-6-ethylpyridazine-5-carboxylic acid,
1-(3-bromophenyl)-1,4-dihydro-4-oxo-6-propylpyridazine-5-carboxylic acid,
1-(2-bromophenyl)-1,4-dihydro-4-oxo-6-butylpyridazine-5-carboxylic acid,
1-(2,4,6-trichlorophenyl)-1,4-dihydro-4-oxo-6-phenylpyridazine-5-carboxylic acid,
1-(4-methylphenyl)-1,4-dihydro-4-oxo-6-phenylpyridazine-5-carboxylic acid,
1-(4-trifluoromethylphenyl)-1,4-dihydro-4-oxo-6-methylpyridazine-5-carboxylic acid,

1-(3-ethoxyphenyl)-1,4-dihydro-4-oxo-6-ethylpyridazine-5-carboxylic acid,
1-butyl-1,4-dihydro-4-oxo-6-propylpyridazine-5-carboxylic acid,
1-(3-cyanophenyl)-1,4-dihydro-4-oxo-6-butylpyridazine-5-carboxylic acid,
1-(2-chloro-4-methylphenyl)-1,4-dihydro-4-oxo-6-phenylpyridazine-5-carboxylic acid,
1-(2-trifluoromethyl-4-chlorophenyl)-1,4-dihydro-4-oxo-6-methylpyridazine-5-carboxylic acid,
1-(2-trifluoromethyl-4-bromophenyl)-1,4-dihydro-4-oxo-6-ethylpyridazine-5-carboxylic acid,
1-(2-chloro-5-trifluoromethylphenyl)-1,4-dihydro-4-oxo-6-ethylpyridazine-5-carboxylic acid,
1-(2-naphthyl)-1,4-dihydro-4-oxo-6-butylpyridazine-5-carboxylic acid, and the sodium, potassium and lithium carboxy salts and the methyl, ethyl, n-propyl, isopropyl and butyl (all isomers) carboxy esters of each of the above compounds and the acid addition salts of all the above listed compounds including the carboxy salts and carboxy esters.

The following is a sequence utilized to prepare the compounds concerned in this invention.

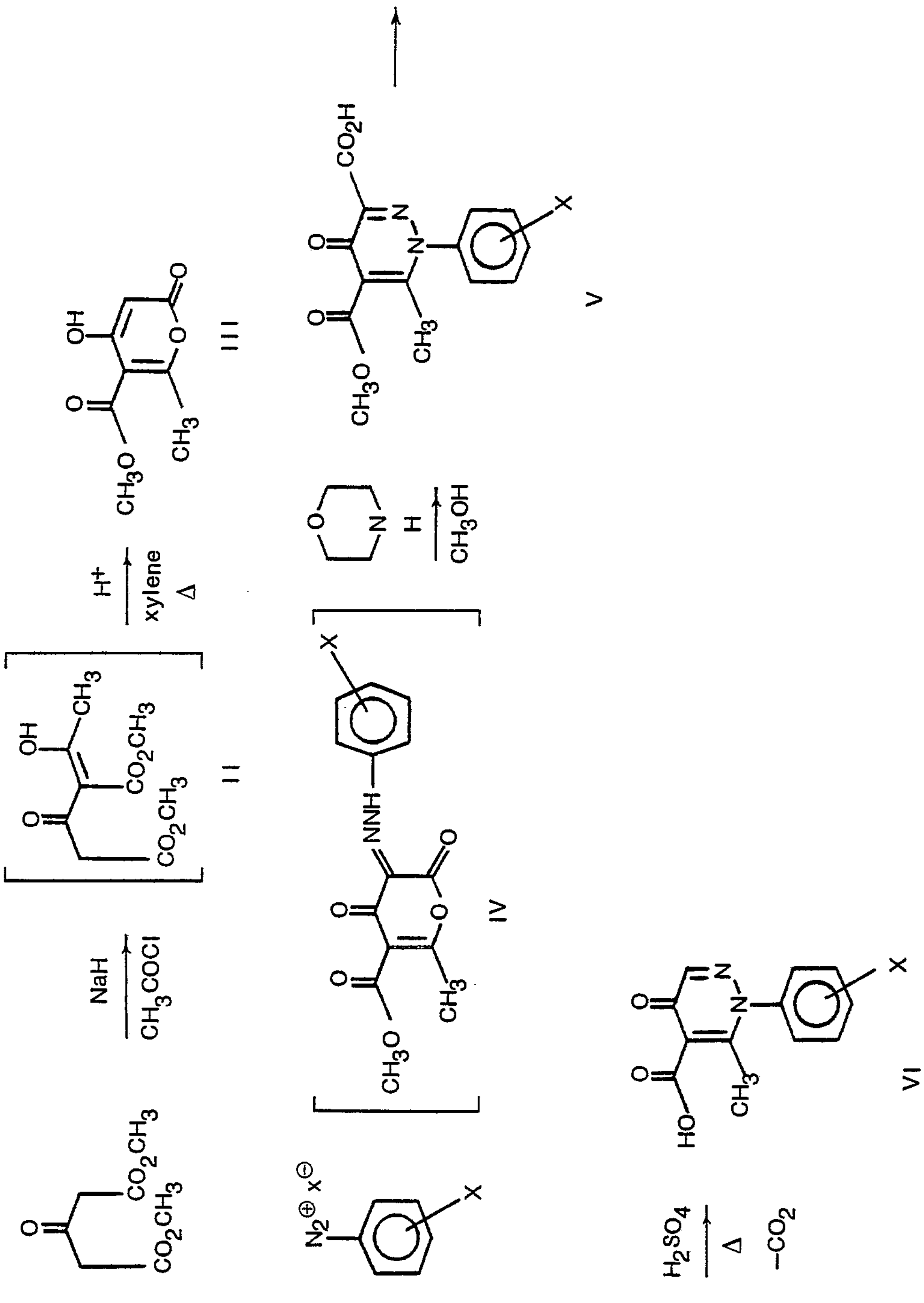


II

III

IV

V

VI

The synthetic sequence outlined above is unique in its ability to produce (VI). No directed syntheses of either of these classes of compounds has been reported in the literature.

The reactions involving the sodium hydride and acetyl chloride can be carried out in an inert solvent at a temperature from about 0° to about 10°C; the reaction of the pyrone with the diazonium salt can be carried out at a temperature from about 0° to about 50°C; and the decarboxylation using sulfuric acid can be carried out in an inert atmosphere from about 20° to 250°C.

In the above reaction sequence a 3-oxoglutarate is first reacted with sodium hydride thereby replacing a hydrogen atom from the active methylene group followed by reaction with acetyl chloride to form the intermediate of Formula II which then rearranges under acid conditions to form the pyrone of Formula III. The pyrone is then reacted with a diazonium salt to form a hydrazone of Formula IV. The hydrazone is then rearranged in the presence of a base to form the dicarboxypyridazine of Formula V which is then decarboxylated under acidic conditions to form the 5-carboxypyridazine of Formula VI.

A more preferred synthetic route to compounds of Formula (I) is outlined below.

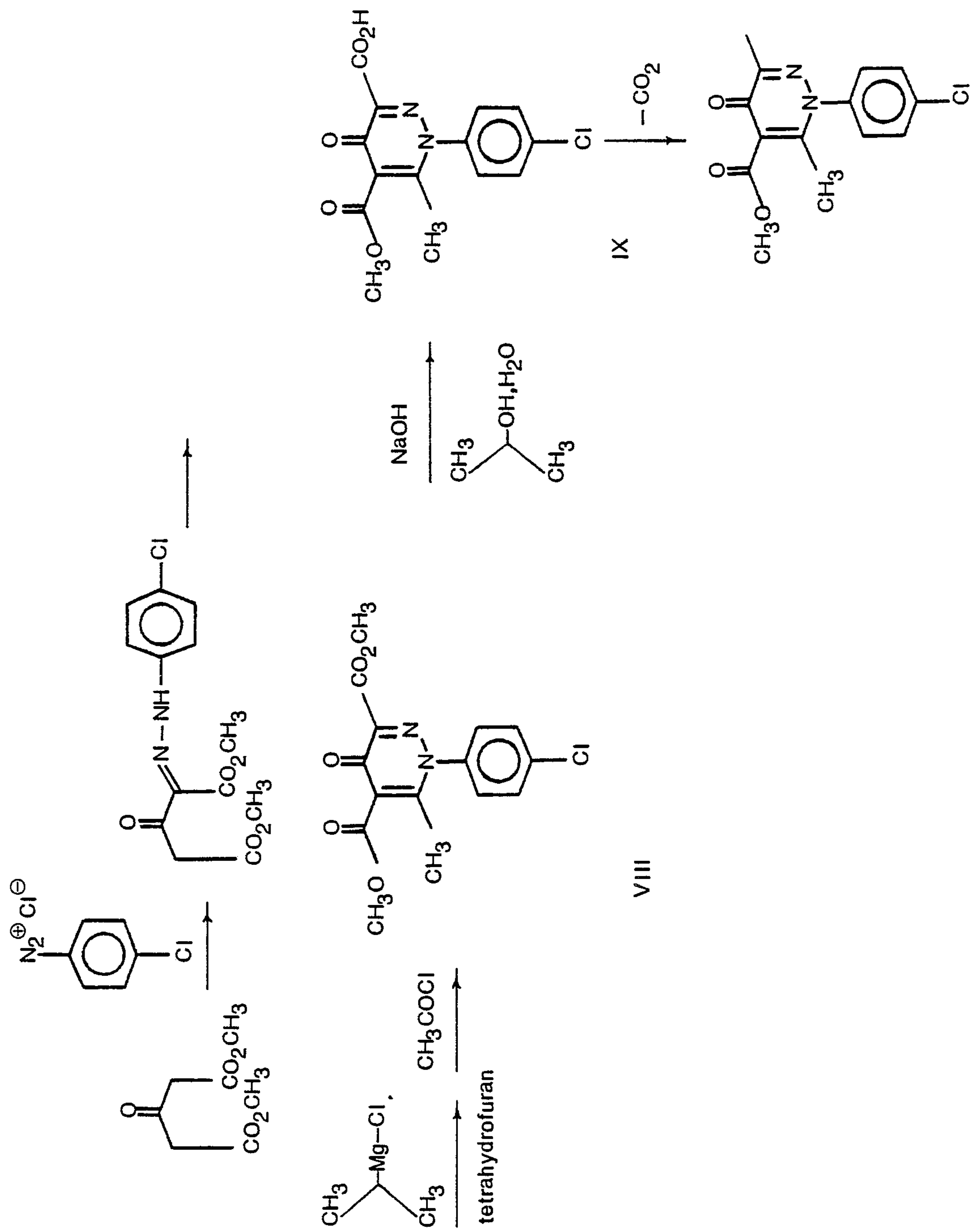


VIII

IX

In this approach the 3-oxoglutarate is first reacted with a diazonium salt to form the hydrazone of Formula VII which is then reacted with isopropyl magnesium chloride followed by reaction with acetyl chloride to form the dicarboxypyridazine ester of Formula VIII which is then hydrolyzed to the corresponding acid of Formula IX. Decarboxylation under strongly acidic conditions as described above will again form the monocarboxy pyridazine of Formula VI.

The following Examples are provided to illustrate the processes for preparing the compounds of Formula I. There may also be used other processes described in the literature for the preparation of analogous compounds such as processes described in Belgian Patent No. 864,704 and its foreign equivalents cited earlier. In the following preparations temperatures are in degrees Celsius and percentages are on a weight basis unless otherwise specified.

Example 1

Synthesis of 6-methyl-5-methoxycarbonyl-4-hydroxy-2-pyrone via dimethyl-2-acetyl-3-oxoglutarate

A 3-liter, three necked, round bottomed flask was equipped with additional funnel, paddle stirrer and thermometer. The flask was charged with 300 ml dry toluene and sodium hydride (50% as a dispersion in mineral oil, 82.8 g, 1.72 moles). The addition funnel was charged with dimethyl-3-oxoglutarate (dimethyl ester of acetone-1,3-dicarboxylic acid, 300 g, 1.72 moles). The flask was cooled to 5° in an ice-water bath. The diester was added dropwise to the sodium hydride slurry, not allowing the reaction temperature to exceed 10°.

Complete addition required 3 hrs. The resulting mixture was stirred 30 min. at 5°. Acetyl chloride (135 g, 1.72 moles) was then added dropwise through the addition funnel, being careful to maintain the pot temperature at 5—10°. After complete addition, the resulting slurry was stirred a further 30 min., then poured slowly into 500 ml water saturated with ammonium chloride. The resulting mixture showed a pH of 6. The layers were separated.

The aqueous phase was extracted with methylene chloride (3 × 100 ml). The combined organic layers were taken to dryness *in vacuo* to leave a yellow oil. Vacuum distillation of this oil (at 0.5 mm Hg) gave fractions boiling from 50—120°. A major fraction, bp 85—110° (97 g) contained the desired acetylated diester, along with some starting material, as inferred by NMR.

The impure acetylated diester (97 g) obtained above, was dissolved in 300 ml dry xylene, along with p-toluenesulfonic acid (100 mg). This mixture was refluxed into a Dean-Stark trap for 12 hr. The resulting dark solution was cooled in an ice-water bath. The desired pyrone crystallizes out as fine needles (28.1 g, 10% yield based on dimethyl 3-oxoglutarate). An analytical sample was crystallized from ethyl acetate. NMR ($CDCl_3$): 5.6 ppm (S, 1H); 4.1 (S, 3H); 2.7 (S, 3H). IR ($CH_2Cl_2$): 5.75μ, 5.95, 6.90, 9.10. mp 104—106°.

Elemental Analysis

Calculated: C: 52.18; H: 4.38
Found: C: 52.30; H: 4.44

Synthesis of 1-(p-Chlorophenyl)-1,4-dihdyro-4-oxo-3-carboxy-5-methoxycarbonyl-6-methylpyridazine

A 250 ml, three necked, round bottomed flask was equipped with addition funnel, paddle stirrer and thermometer. The flask was charged with 50 ml methanol, sodium acetate (16.0 g, 0.198 mole), and the above pyrone (8.0 g, 0.043 mole). p-Chlorobenzenediazonium chloride was prepared on the side by the dropwise addition of sodium nitrite (3.3 g, 0.047 mole) in 10 ml water to a cooled (5°) slurry containing p-chloroaniline (5.6 g, 0.043 mole) in aqueous hydrochloric acid (16.5 ml 12N HCl [0.198 mole] plus 10 ml water). The diazonium chloride solution was added dropwise to the solution containing the pyrone. This addition was carried out during 10 min. with no noticeable exotherm. After complete addition, the resulting orange slurry was stirred for 40 min. at room temperature. Suction filtration gave an orange filter cake which was washed repeatedly with water, then sucked dry during 2 hr. The filter cake thus obtained was placed back into the three necked flask used above. Methanol (200 ml) was added to give a slurry. Morpholine (10.0 g, 0.115 mole) was added in one portion. A mildly exothermic reaction occurred, and a dark, homogeneous solution was obtained. After stirring 10 min., the solution was poured into 300 ml water. This was extracted with methylene chloride (3 × 100 ml). The combined organic extracts were extracted repeatedly with dilute aqueous sodium hydroxide (pH 8). The combined aqueous basic layers were acidified with 6N hydrochloric acid. With cooling and scratching, a solid crystallized from solution. Suction filtration gave the product as a light brown powder (8.2 g, 60% yield based on the pyrone NMR ($CDCl_3$): 7.5 ppm (multiplet, 4H); 4.0 (S, 3H); 2.3 (S, 3H). IR ($CH_2Cl_2$): 5.75μ, 6.22, 6.90. mp 203—204° (dec.). An analytical sample was crystallized from methanol.

Elemental Analysis

Calculated: C: 52.10; H: 3.44; N: 8.68
Found: C: 52.06; H: 3.43: N: 8.89

Synthesis of 1-(p-Chlorophenyl)-1,4-dihyro-4-oxo-5-carboxy-6-methylpyridazine

1-(p-chlorophenyl-1,4-dihydro-4-oxo-3-carboxy-5-methoxy-carbonyl-6-methylpyridazine (2.0 g, 6.2 mmoles) was dissolved in 10 ml, concentrated sulfuric acid, in an atmosphere of dry nitrogen. This was

warmed rapidly to 200° and maintained there for 40 min. The resulting dark solution was cooled, then poured into cold water (50 ml). A brown precipitate formed immediately. Suction filtration and thorough washing with water gave the desired acid (1.0 g 63% yield), pure by NMR.

NRM ($CDCl_3$): 8.5 ppm (S, 1H); 7.6 (AB quartet, 4H); 2.8 ppm (S, 3H). IR (Nujol): 5.81μ. mp 207—208° (dec.). An analytical sample was obtained by crystallization from methanol.

Elemental Analysis

| | |
|---|---|
| Calculated: | C: 54.45; H: 3.43; N: 10.59 |
| Found: | C: 54.45; H: 3.52; N: 10.16 |

Example 2

Synthesis of Dimethyl-2,3-dioxoglutarate, 2-p-chlorophenylhydrazone

A 10-liter widemouthed polyethylene container was fitted with a stirrer paddle and addition funnel. This container was charged with dimethyl-3-oxoglutarate (1 kg, 5.75 moles), methanol (1.5 l), and sodium acetate (1 kg, 12.19 moles). p-Chlorobenzenediazonium chloride (5.75 moles) was generated on the side in seven equal portions, by combining p-chloroaniline (7 × 104 g, 5.75 moles), hydrochloric acid (7 × 314 ml 12N, 26 moles), water (7 × 200 ml) and sodium nitrite (7 × 65.6 g in 100 ml $H_2O$, 6.66 moles). The diazonium salt was added dropwise, rapidly to the reaction kettle. The pH was monitored periodically and maintained at 5 during the course of the reaction by adding sodium acetate *via* a spatula. At the end of the procedure, another 800 g of sodium acetate had been added. The resulting mixture was allowed to stand overnight, then suction filtered and the filter cake washed thoroughly with water. The brick-red filter cake was air dried to give 1.4 kg of the desired product (80% yield). NMR ($CDCl_3$): 7.5 ppm (S, 6H); 3.9 ppm (S, 6H); 3.7 ppm (S, 2H). This compound is known to the literature. See: Bulow and Hopfner, Berichte, *34*, 71 (1901); ibid, *44*, 2835 (1911).

Synthesis of Dimethyl-1-(p-chlorophenyl)-1,4-dihydro-4-oxo-6-methyl-pyridazine-3,5-dicarboxylate

A dry 1-liter, four-necked round bottomed flask was fitted with stirrer, thermometer, nitrogen inlet and rubber septum. The flask was charged with dimethyl-2,3-dioxoglutarate, 2-p-chlorophenylhydrazone (50 g, 0.16 mole) in dry tetrahydrofuran (170 ml). This solution was maintained under an atmosphere of dry nitrogen while cooling to 5°C. Isopropyl magnesium chloride (72 ml, 2.25 N in ethyl ether, 0.16 mole) was added dropwise *via* syringe, maintaining the pot temperature at 5—10°C. After complete addition, the mixture was stirred for 15 min. in the cold, then acetyl chloride (12 ml, 13.0 g, 0.16 mole) was added dropwise, rapidly, keeping the temperature of the reaction mixture below 10°C. The resulting dark solution was allowed to warm to room temperature during 2 hrs. Water (200-ml) was added. This mixture was stirred for 30 min., then extracted with ethyl acetate. The extracts were dried over $MgSO_4$, then filtered and reduced in volume *in vacuo*. The resulting dark oil was dissolved in ethyl ether, and cooled in an ice bath. The diester crystallized out to give 22.1 g yellow powder (42% yield), mp 153—54°C. NMR ($CDCl_3$): 7.6 ppm (multiplet, 4H); 4.0 ppm (S, 6H); 2.3 ppm (S, 3H). IR ($CH_2Cl_2$): 5.75μ, 6.12, 9.15.

From the above-mentioned product the desired compound of Formula (I) is obtained by rearrangement in the presence of a base to form a dicarboxypyridazine which is then decarboxylated under acid conditions as outlined in the penultimate paragraph before Example 1.

Table I below gives the structure, melting point and elemental analysis for some of representative compounds of Formula (I) which were prepared by the processes disclosed above. In Table I the figures first appearing for elemental analysis are those calculated and those immediately thereafter are those found.

TABLE I

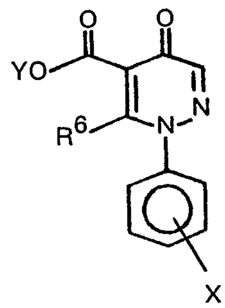

| Compound No. | X | Y | $R^6$ | mp(°C)[(a)] | Elemental Analysis [(a)] C | H | N |
|---|---|---|---|---|---|---|---|
| 1 | 4-Cl | Na | n-Butyl | 157—58° | 58.73<br>58.91 | 4.93<br>4.84 | 9.13<br>9.25 |
| 2 | 4-Cl | Na | n-Propyl | 171—72° | 57.44<br>57.43 | 4.48<br>4.33 | 9.57<br>9.30 |
| 3 | 3,4-diCl | Na | ethyl | 202—03° | 49.86<br>50.12 | 3.22<br>3.27 | 8.95<br>9.32 |
| 4 | H | Na | ethyl | 163—64° | 63.92<br>63.90 | 4.95<br>4.89 | 11.47<br>11.81 |
| 5 | 4-Cl | Na | ethyl | 190—191° | 56.02<br>55.93 | 3.98<br>3.98 | 10.05<br>9.96 |
| 6 | 4-Br | Na | ethyl | 220°(dec) | 48.32<br>48.71 | 3.43<br>3.45 | 8.67<br>8.66 |
| 7 | H | Na | methyl | 204°(dec) | 62.60<br>63.05 | 4.38<br>4.47 | 12.17<br>11.94 |
| 8 | 4-Cl | ethyl | methyl | 175—77° | 57.44<br>57.17 | 4.48<br>4.66 | 9.57<br>9.55 |
| 9 | 4-F | Na | methyl | 211—12° | 58.06<br>57.69 | 3.65<br>3.70 | 11.29<br>11.11 |
| 10 | 4-Cl | methyl | methyl | 140—42° | 56.02<br>55.78 | 3.98<br>4.06 | 10.05<br>10.00 |

TABLE I (continued)

| Compound No. | X | Y | $R^6$ | mp(°C)[a] | Elemental Analysis [a] C | H | N |
|---|---|---|---|---|---|---|---|
| 11 | 3,4-diCl | Na | methyl | 213—14° | 48.18<br>47.52 | 2.69<br>2.72 | 9.37<br>4.74 |
| 12 | 4-Br | Na | methyl | 200—10° (dec) | 46.62<br>46.74 | 2.93<br>2.91 | 9.06<br>9.32 |
| 13 | 4-Cl | Na | methyl | 207—08° | 54.45<br>54.45 | 3.43<br>3.52 | 10.59<br>10.16 |
| 14 | 4-Cl | ethyl | ethyl | 129—131° | 58.73<br>58.61 | 4.93<br>5.12 | 9.13<br>9.26 |
| 15 | 4-Cl | methyl | ethyl | 119—20° | 57.44<br>57.18 | 4.48<br>4.49 | 9.57<br>9.76 |
| 16 | 4-I | Na | ethyl | 185—86° | 43.35<br>41.40 | 3.08<br>2.94 | 7.78<br>7.59 |
| 17 | 2-Cl | Na | ethyl | 174—75° | 56.02<br>55.99 | 3.98<br>3.91 | 10.05<br>10.43 |

[a] Melting Points and Elemental Analyses recorded are for the parent acid of the sodium salts.

The compounds of Formula (I) are particularly useful as chemical hybridization agents in gramineous crops, such as wheat, barley, maize, rice, sorghum, millets, oats, rye, triticale, forage crops and the like. When used as chemical hybridization agents, the compounds induce a selective male sterility without also inducing unacceptable female sterility in the treated plants and without causing unacceptable phytotoxicity towards the treated plants. As used herein, the term male sterility includes both actual male sterility, as evidenced by a lack of male flower parts or by sterile pollen, and functional male sterility, in which the male flower parts are unable to cause pollination. The compounds also cause other plant growth regulatory responses, such as, for example, control of flowering, control of fruiting and inhibition of seed formation in non-cereal species and other related growth regulatory responses.

Where, in the compounds of Formula (I), male sterility is accompanied by some female infertility and/or phytotoxicity, the compounds are still of utility in producing new plant hybrids or in the production of ergot for which see French Published Patent Application No. 2400832.

The compounds of Formula (I) may be used as plant growth regulators in the manner and at doses described in Belgian Patent No. 864,704 and its foreign equivalents listed earlier. The compounds of Formula I may also be formulated, with such changes as are dictated by the solubilities and other characteristics of the compounds in question, as described in the Belgian Patent or its equivalents. The compounds of Formula (I) may be used singly or in admixture or may be used in conjunction with other types of plant growth regulators or with different types of agricultural chemicals as described in the Belgian patent and its equivalents.

Formulations usually include one or more of the compounds of Formula (I), e.g. in an amount from 1—99% by weight, and an agronomically acceptable carrier or diluent.

Of course, reference in the last three paragraphs — and elsewhere in this specification unless the context requires otherwise — to compounds of formula (I) includes the acid addition salts.

In the biological data given later units are expressed in U.S. units, the metric equivalents of which are given below together with other explanataory information.

6 inches = 15.24 cm.
Parts are parts by weight.
Fertilizer (16—25—16) = (16—25—16 by wt. NPK).
1 tsp/gal = 1.3 ml/l.
Isotox® = benzenehexachloride
Feekes' scale is described by W. Feekes, Vers.17
Tech. Tarwe Comm, Groningen, pp 560, 561, 1941.
50 gal/A (acre) = 467.5 l/ha.
2 oz/50 gal = 56.7 g/189.25 l
Dose = dose of active ingredient

| lbs/acre | = | kg/ha | lbs/acre | = | kg/ha |
|---|---|---|---|---|---|
| 1/28 | | 0.009 | 1/2 | | 0.561 |
| 1/64 | | 0.017 | 1 | | 1.121 |
| 1/32 | | 0.035 | 2 | | 2.242 |
| 1/16 | | 0.07 | 4 | | 4.484 |
| 1/8 | | 0.140 | 8 | | 8.968 |
| 1/4 | | 0.280 | | | |

## Chemical Hybridization Activity

The following procedures are used to evaluate the activity of the compounds of the invention for inducing male sterility in cereals.

An awned variety (Fielder) and an awnless variety (Mayo-64) of spring wheat are planted at the rate of 6 to 8 seeds per 6 inch pot containing a sterile medium of 3 parts soil and 1 part humus. The plants are grown under short-day (9 hour) conditions for the first 4 weeks to obtain good vegetative growth before flower initiation. The plants are then moved to long-day (16 hour) conditions which are provided by high intensity lights in the greenhouse. The plants are fertilized at 2, 4, and 8 weeks after planting with a water soluble fertilizer (16—25—16) at the rate of 1 tsp/gal of water, and are frequently sprayed with Isotox® for aphid control and dusted with sulfur for powdery mildew control.

Test compounds are foliarly applied to the awned female plants when these plants reach the flag leaf emergence stage (stage 8 on Feekes' scale). All compounds are applied in various concentrations in an aqueous carrier (containing if necessary, an organic solvent and conventional surfactant). The carrier volume is 50 gal/A (acre) in all cases.

After spike emergence but before anthesis, 4 to 6 spikes per pot are bagged to prevent outcrossing. At the first signs of flower opening, two spikes per pot are cross pollinated, using the approach method, with the awnless male parent. As soon as the seeds become plainly visible, spike length is measured and seeds per spikelet counted in both bagged and cross spikes. Male sterility can then be calculated as percent inhibition of seed set in bagged spikes of treated plants, and female fertility in crossed spikes can be calculated as percent of control seed set. After maturity the seed on crossed spikes can be planted for determination of percent hybridization.

Where applicable, percent sterility, percent fertility, and percent height inhibition are calculated from the following formulas:

a. % Sterility = $(S_c - S_t/S_c) \times 100$
$S_c$ = seeds/spikelet in bagged spikes of control plants.
$S_t$ = seeds/spikelet in bagged spikes of treated plants.

b. % Fertility = $(F_t/F_c) \times 100$
$F_c$ = seeds/spikelet in approach crossed spikes of treated plants
$F_t$ = seeds/spikelet in unbagged spikes of control plants

c. % Height inhibition = $(H_c - H_t/H_c) \times 100$
$H_c$ = Height of control plants
$H_t$ = Height of treated plants

Table II summarizes typical results obtained in the evaluation of compounds of Formula (I).

TABLE II

| Compound No. | Dose (lb/acre) | Male Sterility % | Plant Injury (0—9) |
|---|---|---|---|
| 1 | 1/8 | 0 | 0 |
| | 1/4 | 24.4 | 0 |
| | 1/2 | 74.2 | 0 |
| | 1 | 91.0 | 0 |
| 2 | 2 | 99.1 | 0 |
| 2 | 1/8 | 0 | 0 |
| | 1/4 | 29.4 | 0 |
| | 1/2 | 75.1 | 0 |
| | 1 | 99.1 | 0 |
| | 2 | 100 | 0 |
| 3 | 1/8 | 5.7 | 0 |
| | 1/2 | 57.9 | 0 |
| | 2 | 99.1 | 0 |
| | 8 | 100 | 0 |
| 4 | 1/8 | 3.1 | 0 |
| | 1/2 | 26.6 | 0 |
| | 2 | 46.9 | 0 |
| | 8 | 55.3 | 0 |
| 5 | 1/8 | 77.0 | 0 |
| | 1/2 | 98.0 | 0 |
| | 2 | 100.0 | 0 |
| | 8 | 100.0 | 0 |
| 6 | 1/8 | 20.0 | 0 |
| | 1/2 | 82.0 | 0 |
| | 2 | 99.0 | 0 |
| | 8 | 100.0 | 0 |
| 7 | 1/8 | 0 | 0 |
| | 1/2 | 28.0 | 0 |
| | 2 | 100.0 | 0 |
| | 8 | 100.0 | 0 |
| 8 | 1 | 66.0 | 0 |
| | 2 | 72.0 | 0 |
| | 4 | 100.0 | 0 |
| | 8 | 100.0 | 0 |
| 9 | 1/2 | 1.0 | 0 |
| | 1 | 0 | 0 |
| | 2 | 0 | 0 |
| | 4 | 57.0 | 0 |

Representative compounds of Formula (I) exhibit an improved margin of safety while maintaining high levels of activity, as compared to the known compound 1-(p-chlorophenyl)-1,4-dihydro-4-oxo-6-methylpyridazine-3-carboxylic acid sodium salt, as shown in Table III, below

TABLE III

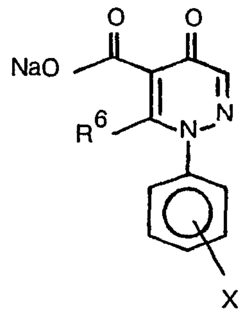

| X | $R^6$ | Dose (lb/acre) | Male Sterility | Plant Injury |
|---|---|---|---|---|
| H | $CH_3CH_2$— | 1/8 | 3.1% | 0 |
| | | 1/2 | 26.6% | 0 |
| | | 2 | 46.9% | 0 |
| | | 8 | 55.3% | 0 |
| 3,4-diCl | $CH_3CH_2$— | 1/8 | 5.7% | 0 |
| | | 1/2 | 57.9% | 0 |
| | | 2 | 99.1% | 0 |
| | | 8 | 100.0% | 0 |
| 4-Cl | $CH_3CH_2$— | 1/8 | 77.0% | 0 |
| | | 1/2 | 98.0% | 0 |
| | | 2 | 100.0% | 0 |
| | | 8 | 100.0% | 0 |
| 4-Br | $CH_3CH_2$— | 1/8 | 20.0% | 0 |
| | | 1/2 | 82.0% | 0 |
| | | 2 | 99.0% | 0 |
| | | 8 | 100.0% | 0 |
| H | $CH_3$ | 1/8 | 0 | 0 |
| | | 1/2 | 28.0% | 0 |
| | | 2 | 100.0% | 0 |
| | | 8 | 100.0% | 0 |
| 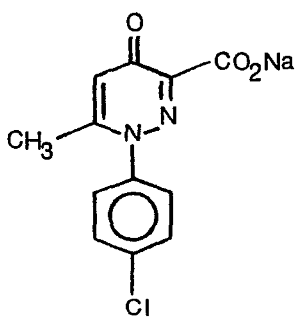 | | 1/4 | 78.0% | 0 |
| | | 1/2 | 96/0% | 0 |
| | | 1 | 100.0% | 1 |
| | | 2 | 100.0% | 5 |

Table IV below gives comparative field test data on winter wheat (variety Newton) using potassium 1-(4′-chlorophenyl)-1,4-dihydro-4-oxo-6-methyl-pyridazine-3-carboxylate (Compound A) with a compound of the invention, potassium 1-(4′-chlorophenyl)-1,4-dihydro-4-oxo-6-ethyl-pyridazine-5-carboxylate (Compound B).

TABLE IV

| Dose (lbs/A) | Male Sterility (%) | Seed Yield (% of Nontreated) | Plant Height (% Inhibition) | Plant Injury (0—9) |
|---|---|---|---|---|
| Compound A | | | | |
| 1/4 | 24 | 87 | 1 | 0 |
| 1/2 | 65 | 61 | 1 | 0 |
| 1 | 99 | 29 | 3 | 0.3 |
| 2 | 100 | 15 | 14 | 1.3 |
| Compound B | | | | |
| 1/2 | 11 | 108 | 0 | 0 |
| 1 | 18 | 88 | 0 | 0 |
| 2 | 39 | 81 | 0 | 0 |
| 4 | 85 | 75 | 0 | 0 |

Table V gives comparative field test data on winter wheat using sodium 1-(4′-chlorophenyl)-1,4-dihydro-4-oxo-6-methyl-pyridazine-3-carboxylate (Compound C) with a compound of the present invention, sodium 1-(4′-chlorophenyl)-1,4-dihydro-4-oxo-6-methyl pyridazine-5-carboxylate (Compound D).

TABLE V

| Compound | Wheat Variety | $ED_{95}$[1] (lb/A) | Seed[2] Yield | Seed[3] Quality | Plant[4] Injury |
|---|---|---|---|---|---|
| C | Abe | 0.6 | 59% | 0.1 | 0 |
| | Hart | 1.5 | 54% | 1.1 | 0 |
| | Centurk | 0.9 | 69% | 0.7 | 0.3 |
| | Cloud | 0.9 | 57% | 2.2 | 0.7 |
| D | Abe | 8.0 | 83% | 0 | 0 |
| | Hart | 15.0 | 73% | 0 | 0 |
| | Centurk | 18.0 | 72% | 0 | 0 |
| | Cloud | 17.0 | 78% | 0 | 0 |

1. dose of active ingredient required to effect 95% male sterility (0.6 lb/A = 0.67 kg/ha, 0.9 lb/A = 1 kg/ha, 1.5 lb/A = 1.7 kg/ha, 8 lb/A = 8.97 kg/ha, 15 lb/A = 16.8 kg/ha, 17 lb/A = 19.06 kg/ha and 18 lb/A = 20.18 kg/ha).
2. As a percent of untreated control.
3. 0—5.0 denotes a normal seed; 5 denotes a very shriveled seed.
4. 0—10.0 denotes no injury; 10 denotes plant kill.

**Claims for the Contracting States: BE CH DE FR GB IT LI NL SE**

1. A compound of Formula (I) below, or an agronomically acceptable acid addition salt thereof,

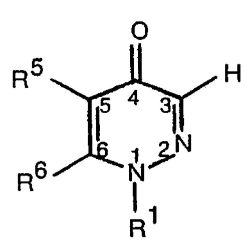

(I)

wherein

$R^1$ is $(C_1—C_4)$alkyl, phenyl or naphthyl each of the last two groups being optionally substituted with up to three of the same or different substituents selected from halogen, trihalomethyl, $(C_1—C_4)$alkoxy, $(C_1—C_4)$alkyl and cyano;

$R^5$ is a carboxy (—COOH) group or agronomically acceptable alkali metal salt thereof, an alkoxycarbonyl (—COOR) group or a substituted carbamoyl (—CONR'R'') group, wherein R represents $(C_1—C_4)$alkyl, R' represents hydrogen or $(C_1—C_4)$alkyl and R'' represents $(C_1—C_4)$alkyl; and

$R^6$ is a $(C_1—C_4)$alkyl group or an optionally substituted phenyl or naphthyl group as defined for $R^1$ above.

2. A compound as claimed in Claim 1, wherein $R^1$ is halo-substituted phenyl, $R^5$ is $—CO_2Na$, $—CO_2K$ or $—CO_2CH_3$ and $R^6$ is methyl or ethyl.

3. A compound as claimed in Claim 2, wherein $R^1$ is 4-chloro- or 4-bromo-phenyl, $R^5$ is $—CO_2Na$ or $—CO_2K$ and $R^6$ is ethyl.

4. A plant growth regulating composition containing at least one compound as claimed in any preceding claim and an agronomically acceptable carrier therefor.

5. A method of regulating the growth of a gramineous crop plant which comprises applying to the growing plant, or to seeds thereof, a growth regulating amount of one or more compounds as claimed in any one of Claims 1—3.

6. A method as claimed in Claim 5 as applied to the production of hybrid seed by using at least one growth regulating compound which induces male sterility without destroying female fertility.

7. A method as claimed in Claim 6 wherein the plant is maize, barley, wheat, sorghum or a forage crop plant.

**Claims for the Contracting State: AT**

1. A plant growth regulating composition containing an active plant growth regulating component and an agronomically acceptable diluent or carrier therefor, wherein the active component comprises at least one compound of Formula (I) below, or an agronomically acceptable acid addition salt thereof,

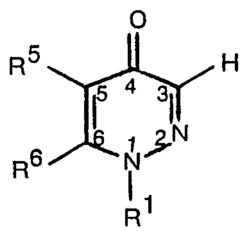

(I)

wherein

$R^1$ is $(C_1—C_4)$alkyl, phenyl or naphthyl each of the last two groups being optionally substituted with up to three of the same or different substituents selected from halogen, trihalomethyl, $(C_1—C_4)$alkoxy, $(C_1—C_4)$alkyl and cyano;

$R^5$ is a carboxy (—COOH) group or agronomically acceptable alkali metal salt thereof, an alkoxycarbonyl (—COOR) group or a substituted carbamoyl (—CONR'R'') group, wherein R represents $(C_1—C_4)$alkyl, R' represents hydrogen or $(C_1—C_4)$alkyl and R'' represents $(C_1—C_4)$alkyl; and

$R^6$ is a $(C_1—C_4)$alkyl group or an optionally substituted phenyl or naphthyl group as defined for $R^1$ above.

2. A composition as claimed in Claim 1, wherein $R^1$ in Formula (I) is halo-substituted phenyl, $R^2$ is $—CO_2Na$, $—CO_2K$ or $—CO_2CH_3$ and $R^6$ is methyl or ethyl.

3. A composition as claimed in Claim 2, wherein $R^1$ is 4-chloro- or 4-bromo-phenyl, $R^5$ is $—CO_2Na$ or $—CO_2K$ and $R^6$ is ethyl.

4. A method of regulating the growth of a gramineous crop plant which comprises applying to the growing plant, or to seeds thereof, a growth regulating amount of a composition as claimed in any one of Claims 1—3.

5. A method as claimed in Claim 4 as applied to the production of hybrid seed by using at least one growth regulating compound which induces male sterility without destroying female fertility.

6. A method as claimed in Claim 5 wherein the plant is maize, barley, wheat, sorghum or a forage crop plant.

**Patentansprüche für die Vertragsstaaten: BE CH DE FR GB IT LI NL SE**

1. Verbindung der folgenden Formel (I) oder für landwirtschaftliche Zwecke verträgliches Säureadditionssalz davon

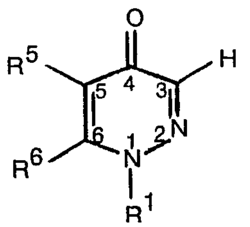

(I)

worin $R^1$ $(C_1—C_4)$-Alkyl, Phenyl oder Naphthyl ist, wobei jede der letzten zwei Gruppen gegebenenfalls mit bis zu drei gleichen oder verschiedenen Substituenten substituiert ist, ausgewählt aus Halogen, Trihalogenmethyl, $(C_1—C_4)$-Alkoxy, $(C_1—C_4)$-Alkyl und Cyano, $R^5$ eine Carboxy-(—COOH)-Gruppe oder ein für landwirtschaftliche Zwecke verträgliches Alkalimetallsalz davon, eine Alkoxycarbonyl-(—COOR)-Gruppe oder eine substituierte Carbamoyl-(—CONR'R'')-Gruppe ist, worin R $(C_1—C_4)$-Alkyl ist, R' Wasserstoff oder $(C_1—C_4)$-Alkyl bedeutet und R'' $(C_1—C_4)$-Alkyl darstellt und $R^6$ eine $(C_1—C_4)$-Alkylgruppe oder eine gegebenenfalls substituierte Phenyl- oder Naphthylgruppe entsprechend der vorstehend Definition für $R^1$ ist.

2. Verbindung nach Anspruch 1, dadurch gekennzeichnet, daß $R^1$ Halogen-substituiertes Phenyl ist, $R^5$—$CO_2Na$, —$CO_2K$ oder —$CO_2CH_3$ ist, und $R^6$ Methyl oder Ethyl darstellt.

3. Verbindung nach Anspruch 2, dadurch gekennzeichnet, daß $R^1$ 4-Chlor- oder 4-Bromphenyl ist, $R^5$—$CO_2Na$ oder —$CO_2K$ ist und $R^6$ Ethyl ist.

4. Pflanzenwachstumsregulierendes Mittel, enthaltend wenigstens eine Verbindung gemäß einem der vorhergehenden Ansprüche und einen für landwirtschaftliche Zwecke verträglichen Träger dafür.

5. Verfahren zum Regulieren des Wachstums einer grasartigen Pflanze, dadurch gekennzeichnet, daß auf die wachsende Pflanze oder auf ihre Saat eine wachstumsregulierende Menge einer oder mehrerer Verbindungen gemäß einem der Ansprüche 1 bis 3 aufgebracht wird.

6. Verfahren nach Anspruch 5, angewendet auf die Erzeugung einer Hybridsaat unter Verwendung wenigstens einer wachstumsregulierenden Menge, die eine männliche Sterilität induziert, ohne die weibliche Fertilität zu zerstören.

7. Verfahren nach Anspruch 6, dadurch gekennzeichnet, daß die Pflanze aus Mais, Gerste, Weizen, Sorghum oder einer Futternutzpflanze besteht.

**Patentansprüche für den Vertragsstaat: AT**

1. Pflanzenwachstumsregulierendes Mittel, enthaltend eine aktive pflanzenwachstumsregulierende Komponente und ein für landwirtschaftliche Zwecke verträgliches Verdünnungsmittel oder einen für landwirtschaftliche Zwecke verträglichen Träger dafür, dadurch gekennzeichnet, daß die aktive Komponente aus wenigstens einer Verbindung der folgenden Formel (I) oder aus einem für landwirtschaftliche Zwecke verträglichen Säureadditionssalz davon besteht

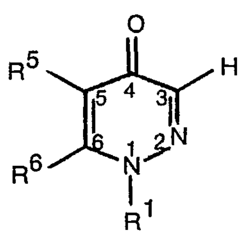

(I)

worin $R^1$ $(C_1—C_4)$-Alkyl, Phenyl oder Naphthyl ist, wobei jede der letzten zwei Gruppen gegebenenfalls mit bis zu drei gleichen oder verschiedenen Substituenten substituiert ist, ausgewählt aus Halogen, Trihalogenmethyl, $(C_1—C_4)$-Alkoxy, $(C_1—C_4)$-Alkyl und Cyano, $R^5$ eine Carboxy-(—COOH)-Gruppe oder ein für landwirtschaftliche Zwecke verträgliches Alkalimetallsalz davon, eine Alkoxycarbonyl-(—COOR)-Gruppe oder eine substituierte Carbamoyl-(—CONR'R'')-Gruppe ist, worin R $(C_1—C_4)$-Alkyl ist, R' Wasserstoff oder $(C_1—C_4)$-Alkyl bedeutet und R'' $(C_1—C_4)$-Alkyl darstellt und $R^6$ eine $(C_1—C_4)$-Alkylgruppe oder eine gegebenenfalls substituierte Phenyl- oder Naphthylgruppe entsprechend der vorstehend Definition für $R^1$ ist.

2. Mittel nach Anspruch 1, dadurch gekennzeichnet, daß $R^1$ in der Formel (I) Halogen-substituiertes Phenyl ist, $R^5$—$CO_2Na$, —$CO_2K$ oder —$CO_2CH_3$ und $R^6$ Methyl oder Ethyl darstellt.

3. Mittel nach Anspruch 2, dadurch gekennzeichnet, daß $R^1$ 4-Chlor- oder 4-Bromphenyl ist, $R^5$—$CO_2Na$ oder —$CO_2K$ ist und $R^6$ Ethyl ist.

4. Verfahren zum Regulierend des Wachstums einer grasartigen Nutzpflanze, dadurch gekennzeichnet, daß auf die wachsende Pflanze oder ihre Saat eine wachstumsregulierende Menge eines Mittels gemäß einem der Ansprüche 1 bis 3 aufgebracht wird.

5. Verfahren nach Anspruch 4, angewendet auf die Erzeugung einer Hybridsaat unter Verwendung wenigstens einer wachstumsregulierenden Menge, die eine männliche Sterilität induziert, ohne dabei die weibliche Fertilität zu zerstören.

6. Verfahren nach Anspruch 5, dadurch gekennzeichnet, daß die Pflanze aus Mais, Gerste, Weizen, Sorghum oder einer Futternutzpflanze besteht.

**Revendications pour les états contractants: BE CH DE FR GB IT LI NL SE**

1. Un composé de formule (I) ci-dessous ou un sel d'addition d'acide convenant en agronomie de celui-ci

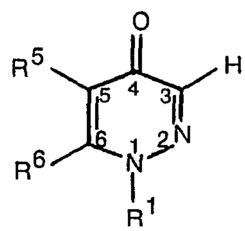

(I)

où

$R^1$ est un alcoyle en $C_1$—$C_4$, un phényle ou un naphtyle, chacun des deux derniers groupes étant éventuellement substitué par jusqu'à 3 substituants semblables ou différents choisis par halogène, trihalogénométhyle, alcoxy en $C_1$—$C_4$, alcoyle en $C_1$—$C_4$ et cyano; $R^5$ est un groupe carboxy (—COOH) ou un sel de métal alcalin convenant en agronomie de celui-ci, un groupe alcoxycarbonyle (—COOR) ou un groupe carbamoyle substitué (—CONR'R''), où R représente un alcoyle en $C_1$—$C_4$, R' représente un hydrogène ou un alcoyle en $C_1$—$C_4$ et R'' représente un alcoyle en $C_1$—$C_4$; et $R^6$ est un groupe alcoyle en $C_1$—$C_4$ ou un groupe phényle ou naphtyle éventuellement substitué comme défini pour $R^1$ ci-dessus.

2. Un composé comme revendiqué dans la revendication 1, dans lequel $R^1$ est un phényle halogéno substitué, $R^5$ est —$CO_2Na$, —$CO_2K$ ou —$CO_2CH_3$ et $R^6$ est un méthyle ou un éthyle.

3. Un composé comme revendiqué dans la revendication 2 où $R^1$ et un 4-chloro- ou un 4-bromophényle, $R^5$ est —$CO_2Na$ ou —$CO_2K$ et $R^6$ est un éthyle.

4. Une composition régulatrice de croissance des plantes contenant au moins un composé comme revendiqué dans l'une quelconque des revendications précédentes et un support convenant en agronomie de celui-ci.

5. Un procédé de régulation de la croissance d'une plante graminée cultivée qui comprend l'application à la plante en développement ou à ses semences d'une quantité régulatrice de la croissance d'un ou plusieurs composés comme revendiqué dans l'une quelconque des revendications 1 à 3.

6. Un procédé comme revendiqué dans la revendication 5 appliqué à la production de semences hybrides par emploi d'au moins un composé régulateur de la croissance qui provoque une stérilité-mâle sans détruire la fertilité-femelle.

7. Un procédé comme revendiqué dans la revendication 6 dans lequel la plante est le maïs, l'orge, le blé, le sorgho ou une plante fourragère cultivée.

**Revendications pour l'Etat contractant: AT**

1. Une composition régulatrice de croissance des plantes contenant un composant actif régulateur de croisssance des plantes et un diluant ou support convenant en agronomie de celui-ci, dans laquelle le composant actif comprend au moins un composé de formule (I) ci-dessous ou un sel d'addition d'acide convenant en agronomie de celui-ci,

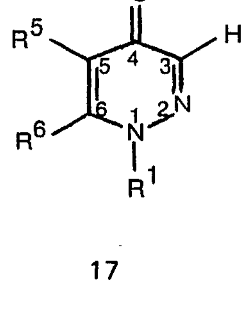

(I)

où

$R^1$ est un alcoyle en $C_1$—$C_4$, un phényle ou un naphtyle, chacun des deux derniers groupes étant éventuellement substitué par jusqu'à 3 substituants semblables ou différents choisis par halogène, trihalogénométhyle, alcoxy en $C_1$—$C_4$, alcoyle en $C_1$—$C_4$ et cyano; $R^5$ est un groupe carboxy (—COOH) ou un sel de métal alcalin convenant en agronomie de celui-ci, un groupe alcoxycarbonyle (—COOR) ou un groupe carbamoyle substitué (—CONR'R''), où R représente un alcoyle en $C_1$—$C_4$, R' représente un hydrogène ou un alcoyle en $C_1$—$C_4$ et R'' représente un alcoyle en $C_1$—$C_4$; et $R^6$ est un groupe alcoyle en $C_1$—$C_4$ ou un groupe phényle ou naphtyle éventuellement substitué comme défini pour $R^1$ ci-dessus.

2. Une composition comme revendiqué dans la revendication 1, dans lequel $R^1$ est un phényle halogéno substitué, $R^5$ est —$CO_2Na$, —$CO_2K$ ou —$CO_2CH_3$ et $R^6$ est un méthyle ou un éthyle.

3. Une composition comme revendiqué dans la revendication 2 où $R^1$ et un 4-chloro- ou un 4-bromophényle, $R^5$ est —$CO_2Na$ ou —$CO_2K$ et $R^6$ est un éthyle.

4. Un procédé de régulation de la croissance d'une plante graminée cultivée qui comprend l'application à la plante en développement ou à ses semences d'une quantité régulatrice de la croissance d'une composition comme revendiqué dans l'une quelconque des revendications 1 à 3.

5. Un procédé comme revendiqué dans la revendication 4 appliqué à la production de semences hybrides par emploi d'au moins un composé régulateur de la croissance qui provoque une stérilité-mâle sans détruire la fertilité-femelle.

6. Un procédé comme revendiqué dans la revendication 5 dans lequel la plante est le maïs, l'orge, le blé, le sorgho ou une plante fourragère cultivée.